# EUROPEAN PATENT APPLICATION

(11) **EP 4 487 904 A1**
(43) Date of publication of application: **08.01.2025**
(21) Application number: 23382687.4
(22) Date of filing: 04.07.2023
(51) Int. Cl.: A61N 5/10

(54) **APPLICATOR FOR INTRAOPERATIVE RADIOTHERAPY TREATMENT**

(71) Applicant: Hospital Clínic de Barcelona, 08036 Barcelona (ES); Fundació de Recerca Clínic Barcelona-Institut d'Investigacions Biomèdiques August Pi I Sunyer, 08036 Barcelona (ES)
(72) Inventor: Cases Copestake, Carla, 08023 Barcelona (ES); Trias Gumbau, Gerard, 08110 Montcada i Reixac (Barcelona) (ES); Herreros Martínez, Antonio, 08290 Cerdanyola del Vallès (Barcelona) (ES); Garcia Causapié, Mario David, 08028 Barcelona (ES); Oses González, Gabriela, 08903 Hospitalet d Llobregat (Barcelona) (ES)
(74) Representative: Clarke, Modet y Cía., S.L.

(57) **Abstract**

Applicator for intraoperative radiotherapy treatment, having a cover (3) which in turn has at the bottom a housing (11) for a shielding disc (1) for intraoperative radiotherapy treatment, a wheel (4) having an attaching rim (13) configured to attach the wheel (4) to a collimator (8) for intraoperative radiotherapy treatment, and attaching means configured to rigidly attach the cover (3) to the wheel (4).

## Description

### Field of the invention

The present invention belongs to the health sector, specifically to oncological surgery, and more specifically to intraoperative electron radiotherapy during oncological surgery for the treatment of breast tumours, although it is also applicable to other types of tumours. The present invention relates to an applicator for attaching a shielding disc to a collimator for intraoperative radiotherapy treatment.

### Background

"Intraoperative electron beam radiotherapy" (IOERT) or shortened "Intraoperative radiotherapy" is a radiotherapy technique applied during oncological surgery to irradiate the tumour bed after the surgical excision of the tumour (mainly breast or gynaecologic cancer, although it could be applied to other types of tumours). For this radiotherapy technique treatment an electron accelerator including a collimator is used to deliver a single dose of radiation therapy to the tumour site.

This process, which only lengthens the surgical intervention by a few minutes, avoids the patient having to return to the hospital to undergo subsequent external radiotherapy treatment or makes it possible for this treatment to be shorter. If this process is not carried out, conventional radiotherapy treatment implies daily visits to the hospital for up to two months.

Currently, the protocol for delivering IOERT after removing the tumour follows the steps:
a) A metal shielding disc at least 1 cm larger than the collimator of the electron accelerator is placed between the tumour bed and the pectoralis fascia for breast tumours. It could be placed between the tumour bed and any fascial tissue in case it was used for treating tumours different from breast tumour ones. The disc has the function to prevent the radiation from reaching the organs located behind the tumour site, mainly lungs, heart or ribs. This metal shielding disc must be placed inside a cover, that avoids hot dose spots related with electron backscatter.
b) The tissue surrounding the tumour is sutured, and the collimator is placed over this tissue.
c) The electron accelerator of the intraoperative radiotherapy equipment is attached to the collimator, and then the prescribed dose is administered according to the clinical case, providing direct irradiation of the tumour bed in a single session, thus avoiding or reducing external radiotherapy sessions.

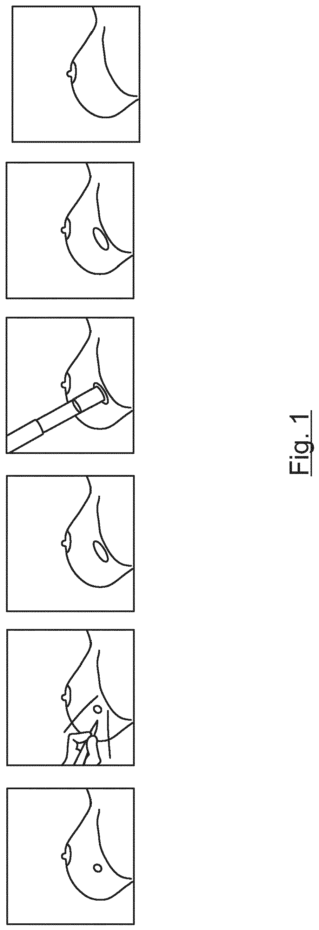

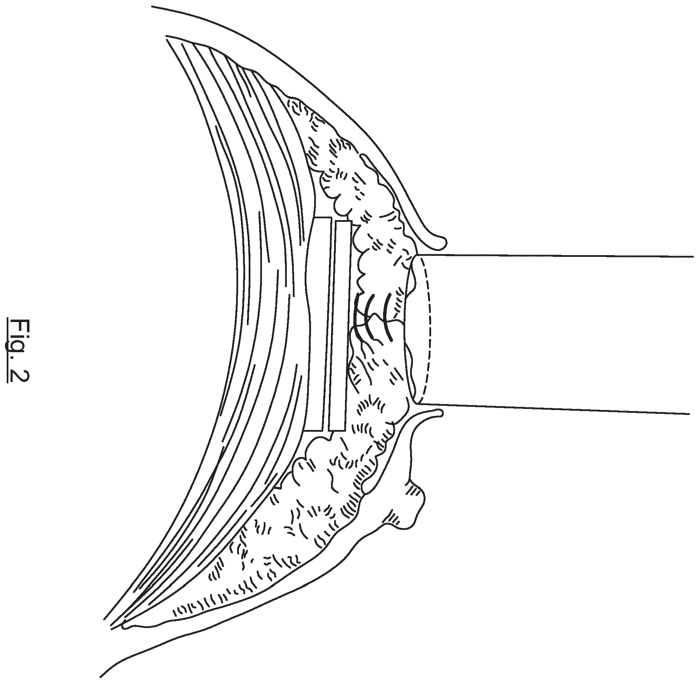

Figure 1 shows a schematic sequential diagram of an oncological surgery for breast cancer including IOERT, and Figure 2 shows a schematic view of a collimator placed on the target tissue, together with a metal shielding disc, according to the current treatment.

Throughout this process, one of the most frequent problems that has the greatest impact on the correct administration of radiotherapy is the relative movement between the metal shielding disc and the collimator. Due to these displacements, on average 20% of the treatment area (ranging from 0% to 60%) falls outside the region protected by the disc, and some amount of misalignment between the shielding disc and the collimator is detected for over 90% of the patients.

This displacement occurs due to relative translations and rotations between the shielding disc and the collimator, and could have adverse effects on adjacent tissues as lungs, heart or ribs, and could produce an under-radiation in the target of the treatment.

So, it is clear that the alignment of the shielding disc with the collimator is a key point in order to provide the best treatment possible for intraoperative radiotherapy. However, the alignment of the shielding disc with the collimator is challenging due to irregularities in the tissue, the disc not being visible during the process, and the inclination of the collimator. Although the shielding disc is sutured to the pectoralis fascia, certain movement of the disc can be expected due to breathing of the patient and gravity.

Currently there are only two known commercial distributors of electron accelerators, having both similar features in terms of the shape of the collimator. Neither of these two commercial distributors provides a solution for the correct alignment between the collimator and the shielding disc. There are currently seven electron accelerators for intraoperative radiotherapy in Spain, at least seventy in Europe and more than a hundred and twenty worldwide.

There is currently no commercial solution to this problem. The only approaches found consist of checking and validating that the shielding disc has not moved prior to irradiation and preventive measures to avoid possible rotation, such as setting a maximum tissue depth difference within the target volume. Alternatively, there are supports that fix the position of the collimator while the accelerator is attached, but none of these solutions address the problem of alignment between the disc and the collimator, and the relative movement between them, especially not during treatment.

It is therefore desirable an applicator for attaching a shielding disc to a collimator for intraoperative radiotherapy treatment preventing the relative movement between the shielding disc and the collimator, and so avoiding the drawbacks of the conventional current intraoperative radiotherapy treatment.

### Description of the invention

The present invention overcomes the drawbacks of the current intraoperative radiotherapy treatment by means of an applicator for intraoperative radiotherapy treatment.

The applicator solidly joins the metal shielding disc with the accelerator collimator. This solid joint between both components prevents any movement (translational or rotational) between them and, therefore, one of the main problems of this type of treatment is solved.

This applicator features a cover that includes a housing at its bottom for a shielding disc used in intraoperative radiotherapy treatment, replacing the current cover of the shielding disc. It also includes a wheel with an attaching rim designed to connect the wheel to a collimator used in intraoperative radiotherapy treatment. Furthermore, it has attaching means that securely fasten the cover to the wheel.

The wheel is attached to the collimator, and therefore it prevents any undesired relative shift and movement between the collimator and the shielding disc. This wheel is designed to be as thin as possible so as not to excessively affect the depth dose profile of the radiotherapy beam.

To prevent unwanted hotspots in the dose distribution, the cover is configured to absorb backscattered radiation reflected by the shielding disc, and so it prevents a radiation overdose due to electron backscatter.

Preferably, the cover is disc-shaped, which facilitates the housing of the shielding disc.

According to a particular embodiment, the attaching means configured to rigidly attach the cover to the wheel comprises at least a screw, which is screwable to corresponding threaded holes on the cover and the wheel. These screws will be manufactured in different sizes to be adapted to different treatments, different patients and different tumours. Besides, the screws are specifically designed so that, when screwed to the wheel and the cover, they do not provide low-dose areas within the volume to be treated.

With accord to different particular embodiments, the cover of the applicator has fixing means to fix the cover to fascial tissue. Preferably, the fixing means comprises a plurality of suturing holes to provide more points of suture, for ease of use by the surgeon and to be able to attach radiochromic films in different holes than the ones used to suture the cover to the fascial tissue. More preferably the suturing holes are placed on the edge of the cover.

In order to ease the extraction of the shielding disc from the housing, the cover may preferably comprise at least a slot placed on the edge of said housing for the shielding disc.

The material of the applicator must provide an electronic density similar to water in order to have a minimal impact on dose distribution within the target volume and needs to be sterilizable. According to different particular embodiments, the applicator is made of polyamide, a medical material that can be sterilized by autokey, has appropriate physical properties and is biocompatible. The polyamide is preferably feinpolyamide PA220, which offers the required resistance while being sterilizable (it can be repeatedly sterilized without degradation) and having a CE certificate.

The applicator object of the present invention allows an acceptable dose distribution within the clinical target volume (CTV) when used. Monte Carlo simulation has been used, using Penelope software to fine tune and validate different designs that would not affect dose distribution in a clinically relevant way. Monte Carlo is considered the Gold standard for the calculation of radiation transport and deposition. Using Penelope a model of the accelerator and the applicator has been generated, and confirmed that the impact of the dose within the CTV is not altered by using the present applicator. Furthermore, two different embodiments have been designed to attach the wheel to the collimator, and can be used, depending on the ease of use and robustness required.

According to a first embodiment, the attaching rim of the wheel is attachable to the collimator outside the walls of the collimator. This first embodiment has a smaller impact within the CTV area but could not be rigid enough during the surgical practice.

With accord to a second embodiment, the attaching rim of the wheel is attachable to the collimator inside the walls of the collimator. This second embodiment diminishes the effective field size and produces small overdosification areas within the CTV. These changes are not relevant clinically and possibly offer a better wheel-collimator union.

The above-disclosed applicator is compatible with the surgical procedure, since it is sterile, easy to handle and robust.

A further advantage of the cover of this applicator is that it flattens the tumour bed, easing the radiotherapy treatment. So, the applicator can avoid the need for a film at the end of the collimator that flattens the tumour bed prior to irradiation. Due to the continuous step of the screwing system, the cover can be progressively screwed and press the underlying tissue, thus substituting the film currently used to flatten the tumour bed avoiding irregularities. Furthermore, the fact that it is rigid ensures a more efficient role in that regard.

At clinical level, with this solution, unnecessary radiation to nearby organs located beyond the disc could be avoided, or at least reduced. This applicator could reduce the dose outside the area to be irradiated by an average of 20%, with cases reaching a reduction of more than 50%. In addition, it would ensure a correct irradiation of the tumour site as it avoids not only displacements but also rotations between the disc and the collimator.

The features, functions and advantages that have been discussed can be achieved independently in various embodiments or may be combined in yet other embodiments. Further details can be seen with reference to the following description and drawings.

### Brief description of the drawings

Next, in order to facilitate the comprehension of this disclosure, in an illustrative rather than limitative manner a series of embodiments with reference to a series of figures shall be made below.
Figure 1 shows a schematic sequential diagram of a current oncological surgery for breast cancer including intraoperative radiotherapy treatment by means of a collimator.
Figure 2 shows a schematic view of a collimator placed on a target tissue, according to the current intraoperative radiotherapy treatment, together with a metal shielding disc provided to prevent the radiation from reaching organs located behind the tumour site.

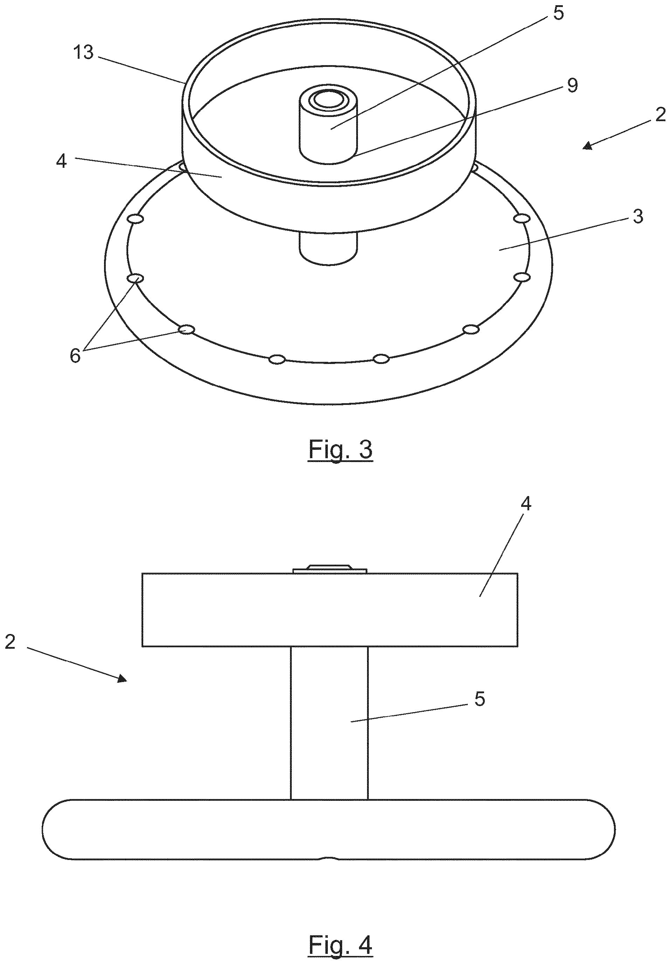

Figure 3 is a perspective view of a particular embodiment of an applicator for intraoperative radiotherapy treatment of the present invention.
Figure 4 is an elevation view of the applicator of figure 3.

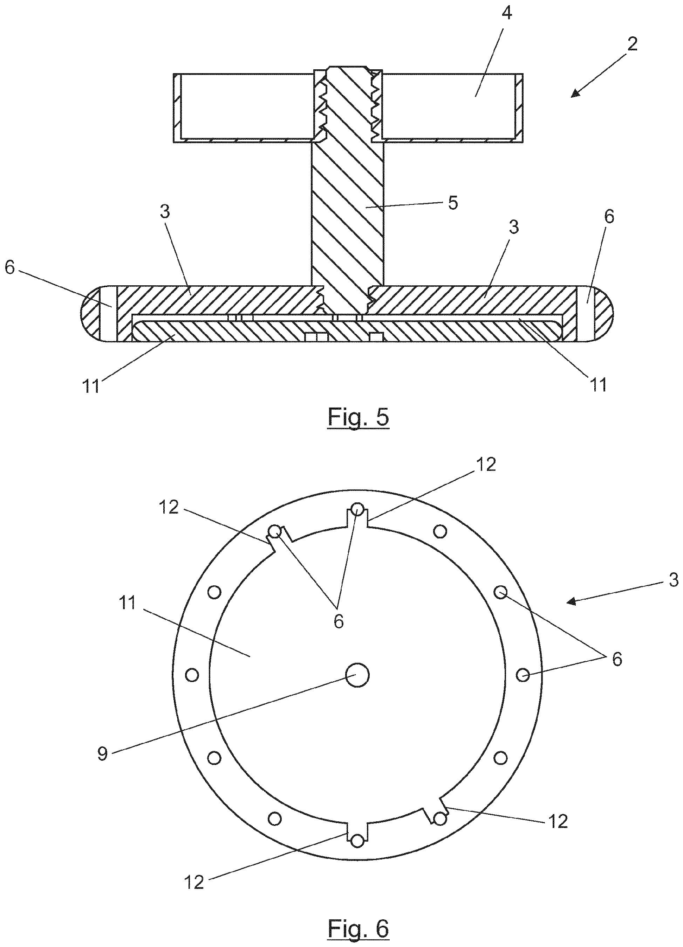

Figure 5 is an elevation section view of the applicator of figures 3 and 4.
Figure 6 is a bottom plan view of the applicator of figures 3, 4 and 5.

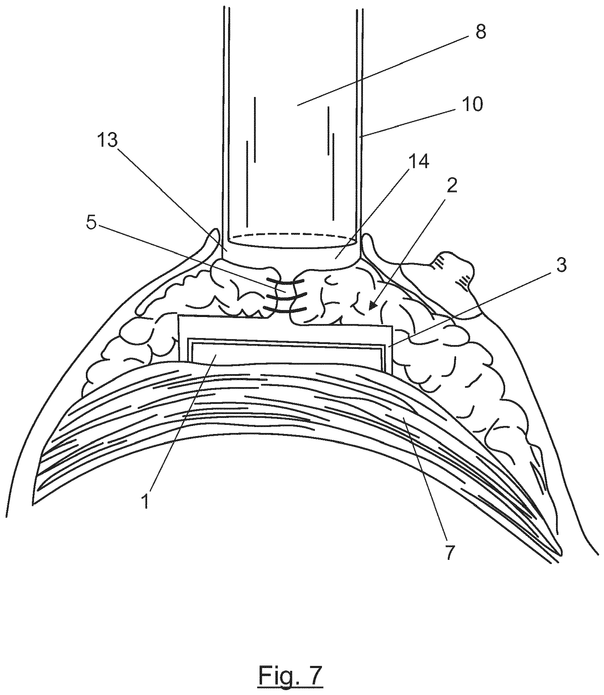

Figure 7 shows a schematic view of a collimator placed on a target tissue, using an applicator according to the present invention for intraoperative radiotherapy treatment.
Figure 8 shows a schematic view of an applicator of the present invention according to first attachment means to a collimator.
Figure 9 shows a schematic view of an applicator of the present invention according to second attachment means to a collimator.
Figure 10a shows a diagram of the applicator depicting three simulated depths (0,1 cm, 0,7 cm and 1,4 cm). Figures 10b, 10c and 10d show comparative diagrams of dose profiles with and without the applicator of the present invention (solid and dotted lines respectively) for 8 MeV, 10b showing dose profiles at 0,1 cm, 10c showing dose profiles at 0,7 cm and 10 d showing dose profiles at 1,4 cm.

These figures refer to the following set of elements:
- 1.: shielding disc
- 2.: applicator
- 3.: cover of the applicator
- 4.: wheel of the applicator
- 5.: screw of the applicator
- 6.: suturing holes of the cover
- 7.: fascial tissue
- 8.: collimator
- 9.: threaded holes
- 10.: walls of the collimator
- 11.: housing of the cover for a shielding disc
- 12.: slot at the edge of the housing for the shielding disc
- 13.: attaching rim of the wheel

### Detailed description

The present disclosure refers to an applicator (2) for intraoperative radiotherapy treatment.

As it can be seen in the figures, the applicator (2) has a cover (3), which comprises at the bottom thereof a housing (11) for a shielding disc (1) used for intraoperative radiotherapy treatment. The applicator (2) has additionally a wheel (4) with an attaching rim (13) to attach said wheel (4) to a collimator (8) used for intraoperative radiotherapy treatment. The applicator (2) has further attaching means configured to rigidly attach the cover (3) to the wheel (4).

The cover (3) of the applicator (2) is configured to absorb backscattered radiation reflected by the shielding disc (1), in order to prevent unwanted radiation during intraoperative radiotherapy treatment.

As it can be shown in the figures, the cover (3) of the applicator (2) is preferably disc-shaped, which facilitates the housing of the shielding disc (1).

According to a particular embodiment, the attaching means of the applicator (2) for rigidly attaching the cover (3) to the wheel (4) comprises at least a screw (5), which is screwable to corresponding threaded holes (9) on the cover (3) and the wheel (4) of the applicator (2).

With accord to particular embodiments of the invention, the cover (3) of the applicator (2) has fixing means to fix the cover (3) to fascial tissue (7) of a patient, in which intraoperative radiotherapy treatment is carried out. Preferably, this fixing means has a plurality of suturing holes (6), and more preferably these suturing holes (6) are placed on the edge of the cover (3).

The cover (3) may preferably comprise at least a slot (12) placed on the edge of the housing (11) for the shielding disc (1), in order to ease the extraction of the shielding disc (1) from the housing (11). Several slots (12) placed along the edge of the housing (11) will make the extraction of the shielding disc (1) even easier.

According to different particular embodiments, the applicator (2) may be made integrally of polyamide, being the polyamide preferably feinpolyamide PA220.

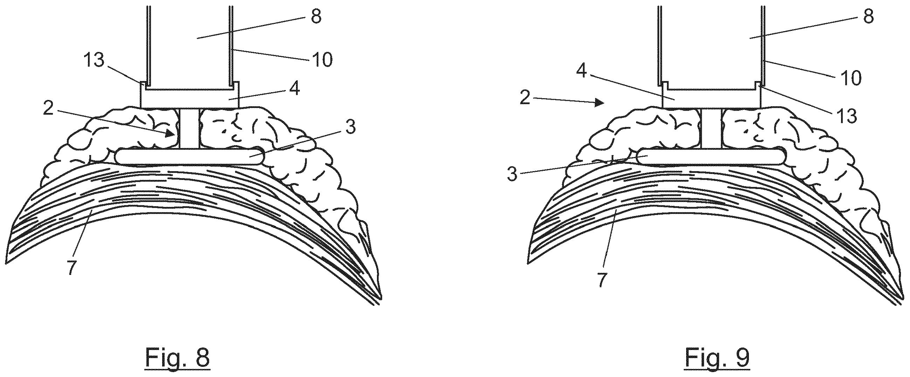

There are different embodiments to attach the wheel (4) of the applicator (2) to the collimator (8) that will emit the radiotherapy beam. According to a first embodiment, the attaching rim (13) of the wheel (4) is attachable to the collimator (8) outside the walls of said collimator (8), as shown if figure 8. This first embodiment has a smaller impact within the CTV area but could not be rigid enough during the surgical practice. With accord to a second embodiment, the attaching rim (13) of the wheel (4) is attachable to the collimator (8) inside the walls of said collimator (8), as shown in figure 9. This second embodiment diminishes the effective field size and produces small overdosification areas within the CTV. These changes are not relevant clinically and possibly offer a better wheel-collimator union.

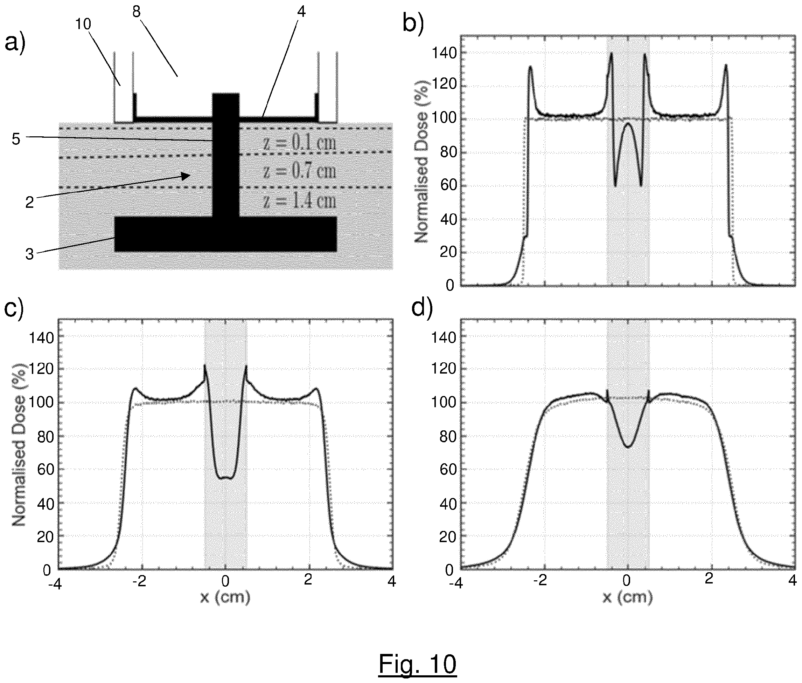

Figure 10a shows a diagram of the applicator (2) depicting three simulated depths (0,1 cm, 0,7 cm and 1,4 cm). Figures 10b, 10c and 10d show comparative diagrams of dose profiles with and without the applicator (2) of the present invention (solid and dotted lines respectively) for 8 MeV, 10b showing dose profiles at 0,1 cm, 10c showing dose profiles at 0,7 cm and 10 d showing dose profiles at 1,4 cm. Grey band of the diagrams represents the inner part of the screw (5) of the applicator (2) (where the infradosification is not a problem, as there is no tissue inside of it). These diagrams refer to the second embodiment disclosed above (attaching rim (13) of the wheel (4) attached to the collimator (8) inside the walls of said collimator (8)). The horns depicted next to the ends of the collimator (8) are too small to be a concern, but they are not present if first embodiment (attaching rim (13) of the wheel (4) attached to the collimator (8) outside the walls of said collimator (8)) is used.

During the design process of the applicator (2) the sizes of the different parts are fine tuned to assure that the low dose regions fall within the screw and did not present a infradosification risk within the CTV. This fine tuning includes a precise combination of screw (5) walls and thickness, so that both the horns and the infradosification fall within the screw area, so that no impact is to be seen in the CTV. At the same time, all parameters must allow a robust enough prototype to hold the forces and tensions of the surgical process.

It has also been verified that the electron backscatter related to the shielding disc (1) is correctly absorbed in the cover (3) of the applicator (2).

Further, the different parts of the applicator (2) can be manufactured to be adaptable to all collimator (8) sizes available (ranging from 3 cm to 9 cm). The design of different sizes of wheels (4) is considered, so that the wheels (4) are adaptable to collimator (8) diameters of 3, 4, 5, 6, 7, 8 and 9 cm, compatibles with the accelerator available collimator (8) sizes. The dimensions of the wheels (4) only vary in the diameter of the flat area, while the screw (5) and walls remain identical for all wheels (4). This allows them to be used for different cases. Besides, the design of different sizes of covers (3) is considered, so that the covers (3) are adaptable to all shielding disc (1) sizes available (ranging from 4 cm to 9 cm). As for the wheels (4), the design of different sizes of covers (3) is considered, so that the covers (3) are adaptable to available shielding disc (1) sizes of 4, 5, 6, 7. 8 and 9 cm. The only dimension that varies for different cover (3) sizes is the total diameter of the flat surface of the cover (3), so the walls, number of holes (6), screw (5) area and depth remain the same for all covers (3), allowing them to be used for different cases. Therefore, different sizes of collimator (8), shielding disc (1), screws (5), covers (3) and wheels (4) can be combined. So, all parts can be printed and combined properly using the sizes required for each specific treatment.

The applicator (2) object of the present invention is adaptable to a wide range of treatment depths according to the energies and prescription features available for the machine, including energies of 4, 6, 8 and 10 MeV, which provides treatment ranges from 6,5 to 22 mm. With the available energies of the accelerator, tumour beds with depths ranging from 6,5 mm to 22mm can be treated. A set of screws (5), with the flat region of the screw (5) ranging from 6,5 mm to 22 mm has been designed. This set consists of six screws (5) with lengths of 6 mm, 9 mm, 12 mm, 15 mm, 18 mm and 22 mm. This allows a continuous treatment depth, considering that screws (5) can be partially coiled up to half its's depth.

The features, functions and advantages that have been discussed can be achieved independently in various embodiments or may be combined in yet other embodiments. Further details can be seen with reference to the following description and drawings.

## Claims

1. Applicator for intraoperative radiotherapy treatment, **characterized in that** it comprises
- a cover (3) comprising at the bottom a housing (11) for a shielding disc (1) for intraoperative radiotherapy treatment, said cover configured to absorb backscattered radiation reflected by the shielding disc (1),
- a wheel (4) comprising an attaching rim (13) configured to attach the wheel (4) to a collimator (8) for intraoperative radiotherapy treatment,
- and attaching means configured to rigidly attach the cover (3) to the wheel (4).

2. Applicator for intraoperative radiotherapy treatment, according to claim 1, wherein the attaching means configured to rigidly attach the cover (3) to the wheel (4) comprises at least a screw (5) screwable to corresponding threaded holes (9) placed on the cover (3) and the wheel (4).

3. Applicator for intraoperative radiotherapy treatment, according to claim 2, wherein the distance between the cover (3) and the wheel (4) is adjustable.

4. Applicator for intraoperative radiotherapy treatment, according to any of claims 1 to 3, wherein the cover (3) is disc-shaped.

5. Applicator for intraoperative radiotherapy treatment, according to any of claims 1 to 4, wherein the cover (3) of the applicator (2) comprises fixing means configured to fix the cover (3) to fascial tissue (7).

6. Applicator for intraoperative radiotherapy treatment, according to claim 5, wherein the fixing means configured to fix the cover (3) to fascial tissue (7) comprises a plurality of suturing holes (6).

7. Applicator for intraoperative radiotherapy treatment, according to claim 6, wherein the suturing holes (6) are placed on the edge of the cover (3).

8. Applicator for intraoperative radiotherapy treatment, according to any of claims 1 to 7, wherein the cover (3) comprises at least a slot (12) on the edge of the housing (11) for the shielding disc (1).

9. Applicator for intraoperative radiotherapy treatment, according to any of claims 1 to 8, wherein the applicator (2) is made of polyamide.

10. Applicator for intraoperative radiotherapy treatment, according to claim 9, wherein the polyamide is feinpolyamide PA220.

11. Applicator for intraoperative radiotherapy treatment, according to any of claims 1 to 10, wherein the attaching rim (13) of the wheel (4) is attachable to the collimator (8) outside the walls (10) of the collimator (8).

12. Applicator for intraoperative radiotherapy treatment, according to any of claims 1 to 10, wherein the attaching rim (13) of the wheel (4) is attachable to the collimator (8) inside the walls (10) of the collimator (8).
